Europäisches Patentamt

European Patent Office

Office européen des brevets

(19)

(11) **EP 1 140 444 B1**

(12) **FASCICULE DE BREVET EUROPEEN**

(45) Date de publication et mention
de la délivrance du brevet:
**10.12.2003 Bulletin 2003/50**

(21) Numéro de dépôt: **99961996.8**

(22) Date de dépôt: **17.12.1999**

(51) Int Cl.⁷: **B27K 5/00**, B27K 5/06

(86) Numéro de dépôt international:
**PCT/BE99/00164**

(87) Numéro de publication internationale:
**WO 00/040382 (13.07.2000 Gazette 2000/28)**

(54) **UTILISATION D'UNE COMPOSITION ENZYMATIQUE ET PROCEDE POUR L'AFFINAGE DU BOIS VERT**

VERWENDUNG EINER ENZYMATISCHEN ZUSAMMENSETZUNG UND VERFAHREN ZUR AFFINAGE VON FRISCHEM HOLZ

USE OF AN ENZYMATIC COMPOSITION AND PROCESS FOR REFINING LIVE WOOD

(84) Etats contractants désignés:
**AT BE CH CY DE DK ES FI FR GB GR IE IT LI LU MC NL PT SE**

(30) Priorité: **31.12.1998 EP 98124852**
**25.06.1999 BE 9900444**

(43) Date de publication de la demande:
**10.10.2001 Bulletin 2001/41**

(73) Titulaire: **Ets Robert Stiernon S.A./N.V.**
**7850 Petit-Enghien (BE)**

(72) Inventeur: **HENRY, Olivier**
**B-1300 Wavre (BE)**

(74) Mandataire: **Vandeberg, Marie-Paule L.G.**
**Office Kirkpatrick S.A.,**
**32, Avenue Wolfers**
**1310 La Hulpe (BE)**

(56) Documents cités:
**EP-A- 0 001 540          DD-A- 292 864**
**DE-C- 946 845            FR-A- 2 421 039**
**FR-A- 2 705 607          US-A- 5 531 898**

• **DATABASE WPI Section Ch, Week 197751 Derwent Publications Ltd., London, GB; Class C03, AN 1977-91378Y XP002138537 & JP 50 024405 A (JAPAN PLYWOOD TECH), 15 mars 1975 (1975-03-15)**

**Description**

La présente invention concerne l'utilisation d'une composition enzymatique et d'un bain aqueux pour l'affinage ou la maturation de bois verts, ainsi qu'un procédé utilisant cette composition enzymatique ou ce bain.

INTRODUCTION

L'utilisation de bois comme matériau en construction, menuiserie ou ébénisterie suppose un séchage préalable afin de stabiliser celui-ci dimensionnellement. Pour la tonnellerie, il existe en outre des exigences organoleptiques.

Pour la plupart des applications industrielles, ce séchage se fait en séchoir à température et humidité contrôlées.

Pour d'autres applications notamment dans les cas où le bois sera par la suite en contact avec des aliments, le séchage se fait naturellement, à l'extérieur, sur des durées assez longues, dans le but d'éliminer certains composés indésirables. La durée de ce séchage naturel ou vieillissement est variable en fonction de l'utilisation finale. Ce séchage peut atteindre 2 ans pour obtenir de bonnes qualités organoleptiques.

Dans le cas de séchage naturel, le bois est colonisé par une flore fongique qui peut transformer certains composés du bois. Ces transformations du bois ne sont cependant pas contrôlables. Les bois séchés naturellement présentent une grande variabilité de qualité.

Par contre, avec un séchage artificiel en séchoir, ces transformations n'ont pas lieu et le bois ainsi séché n'est pas approprié pour des applications alimentaires car il ne permet pas d'éliminer certaines molécules indésirables (telles que des molécules toxiques
ou amères).

ETAT DE LA TECHNIQUE

EP 0 001 540 A concerne l'utilisation de micro-organismes ou d'enzymes (pectinase ou cellulase) dans le but de modifier la structure de bois destiné à la décoration, en contrôlant la température, la teneur du bois en eau, la teneur en $O_2$ et en $CO_2$ dans l'environnement du bois. Dans ce procédé, le bois reçoit un pré-traitement avant l'inoculation, et un post-traitement pour mettre fin au processus microbiologique, suivis d'un séchage.

Ce procédé complexe ne fait pas appel à une immersion du bois dans une préparation enzymatique.

DD 292 864 A concerne l'amélioration de l'imprégnabilité de l'aubier de bois résineux au moyen d'une préparation enzymatique (pectinase, cellulase et hemicellulase), en même temps qu'une préparation minérale aqueuse destinée à la protection superficielle de ce bois.

Ce procédé ne concerne pas le duramen du bois, ni d'autres essences que les résineux, ni une application alimentaire.

FR 2 421 039 A concerne le conditionnement de bois en milieu solide, au moyen de micro-organismes, en vue de faciliter la fragmentation puis l'agglomération des éléments du bois.

Ce procédé ne fait pas appel à une immersion du bois, ni à une préparation enzymatique et ne concerne pas une application alimentaire.

FR 2 705 607 A concerne l'utilisation de micro-organismes présentant des activités α-amylase et β-glucosidase en vue d'améliorer les qualités organoleptiques du bois de chêne destiné à la tonnellerie. Le processus microbiologique est conduit à l'état solide au moyen d'un levain fongique. Le processus censé reproduire les effets d'un séchage/affinage naturel, ne spécifie ni la durée de mise en contact des micro-organismes avec le bois, ni la température, ni la teneur en micro-organismes. Il ne décrit pas un procédé d'immersion du bois, ni une préparation enzymatique et il ne s'applique qu'à des essences de chêne.

DESCRIPTION DE L'INVENTION

La présente invention a pour but de fournir un procédé d'affinage du bois vert qui permet, après son application, un séchage notablement raccourci du bois. Ce procédé est approprié à des applications alimentaires et conserve la stabilité dimensionnelle du bois traité.

L'invention a pour objet l'utilisation d'une composition enzymatique pour le séchage du bois vert comprenant principalement des enzymes de la classe des cellulases, ainsi que d'un bain aqueux pour l'affinage du bois vert comprenant une activité endocellulase comprise entre $1.10^6$ et $100.10^6$ ECU (de préférence entre $3.10^6$ et $30.10^6$ ECU) par m3 de bois à traiter, une activité xylanase comprise entre $0,6.10^6$ et $60.10^6$ BXU (de préférence entre $2.10^6$ et $20.10^6$ BXU) par m3 de bois à traiter, une activité β-mannanase comprise entre $0,4.10^6$ et $40.10^6$ MNU (de préférence entre $1.10^6$ et $10.10^6$ MNU) par m3 de bois à traiter et une activité α-amylase comprise entre $1.10^6$ et $100.10^6$ αTU (de préférence entre $3.10^6$ et $30.10^6$ αTU) par m3 de bois à traiter.

L'invention a également pour objet un procédé d'affinage du bois vert, préalable au séchage, comportant une étape de mise en contact du bois avec un bain aqueux comportant la composition suivant l'invention.

Des modes de réalisations particuliers de l'invention sont décrits dans les revendications dépendantes.

La composition enzymatique utilisée peut comprendre des enzymes de type exocellulaires ou endocellulaires. Les enzymes peuvent être d'origine bactérienne, fongique ou de toute autre origine possible.

DESCRIPTION DETAILLEE DE L'INVENTION

Le bois à traiter, dont la teneur en humidité a été amenée idéalement entre 25 et 45% par un passage rapide au séchoir, est plongé dans un bain contenant de l'eau ou tout autre solvant approprié et des quantités variables d'enzymes principalement de la classe des hydrolases (classe 3 de l'union internationale de biochimie (IUB)) choisies parmi les enzymes de type cellulase (également connues sous le nom de glucosidases) (sous-classe 3.2 de la classification IUB). En outre, on peut ajouter des enzymes de type lipases (sous-classe 3.1.), peroxydases (sous-classe 1. 11.1), etc., et les mélanges de ces enzymes. Parmi les cellulases utilisables pour la présente invention, on peut notamment citer les cellobiohydrolases, les endoglucanases, les β-glucosidases, les hémicellulases, les α-amylases, et les xylanases, les endocellulases, les β-mannases, etc., sans perdre de vue que les enzymes présentent souvent des activités secondaires à côté de leur activité principale.

En fonction de l'utilisation de cellulases neutres ou acides, le pH du bain est ajusté entre 3 et 8, de préférence entre 4 et 7. La température du bain est maintenue constante entre 20 et 70°C, de préférence entre 30 et 50°C. Après ce traitement par immersion pouvant durer de 30 minutes à 2 semaines, de préférence de 1 heure à 1 semaine, en fonction de l'intensité de l'effet désiré, du mélange d'enzymes utilisé, du volume à traiter, du volume du bain, de la nature du bois traité et de la température et du pH du bain, le bois est alors mis au séchoir à température et humidité contrôlées pour réaliser un séchage classique permettant d'atteindre un taux d'humidité de 15 à 20 % pour un usage en tonnellerie, au départ d'un bois ayant un taux d'humidité compris entre 25 et 45%, de préférence entre 30 et 40%, avant la mise en contact avec la préparation enzymatique.

Il s'est avéré que le procédé suivant l'invention permet une hydrolyse des ellagitanins solubles naturellement présents dans le bois, d'une partie des ellagitanins liés aux polysaccharides pariétaux, ainsi que de diverses molécules aromatiques (telles que l'acide digallique, ou les coumarines hétérosidiques) qui sont transformées, par le procédé suivant l'invention, en molécules gustativement neutres.

Le procédé suivant l'invention permet donc d'améliorer l'impression gustative des extraits de bois en éliminant une partie significative des composés indésirables (comme certaines composés phénoliques) et en hydrolysant des formes jugées amères ou astringentes. Ceci est particulièrement important pour les bois devant par la suite entrer en contact avec des aliments (typiquement le bois de tonnellerie).

Ce procédé modifie de façon superficielle l'ultrastructure du bois. Par microscopie électronique, on note une diminution de la paroi cellulaire sur 1 mm. Sur 1 à 3 mm, les pores des cellules sont ouverts. Le procédé fait sentir ses effets jusqu'à environ 5 mm de profondeur.

La présente invention a encore comme avantage de fournir un procédé rapide de vieillissement du bois tout en respectant l'ensemble des réactions physico-chimiques liées au séchage naturel du bois.

De plus, l'invention a l'avantage de fournir un procédé reproductible d'affinage du bois et de permettre une meilleure homogénéité de la qualité des bois traités entre des espèces de bois différentes ou de provenance différente ou entre les différentes parties d'un même arbre. On retrouve donc une meilleure constance dans les propriétés physico-chimiques (mécaniques et aromatiques notamment) du bois traité selon l'invention par rapport au bois séché de manière naturelle et cela de façon indépendante des facteurs climatiques.

Le procédé suivant l'invention permet également, par un choix approprié d'enzymes présentant des activités plus particulières, de conférer au bois des propriétés particulières telles que l'expression d'odeurs ou arômes particuliers.

MATERIEL ET METHODES

1. Origine des échantillons de bois

Les échantillons de chêne sont constitués par le bois de coeur duraminisé d'arbres âgés de 110 à 150 ans, issus de massifs forestiers homogènes et convenablement entretenus. Seul le quart inférieur du tronc, constituant généralement le bois de tonnellerie est utilisé pour l'étude. Les échantillons traités par la composition enzymatique selon l'invention sont simplement séchés au séchoir (1 mois, 40° C, avec ventilation). Les différents échantillons pris au hasard sont rabotés puis réduits en sciure par broyage dans l'azote liquide, avant d'être tamisés pour ne conserver que les particules de dimension inférieure à 250 μm. Les échantillons sont conservés après lyophilisation pour être analysés dans un délai de 2 mois. L'espèce étudiée est *Quercus petraea* (Allier, France).

## 2. Réalisation des extraits et contrôle de leur composition

1g de sciure (250 μm) est extrait par 100 ml de solvant (acétone/eau 7 :3 en volume) pendant 12 h à température ambiante sur table d'agitation; l'extrait obtenu est ensuite filtré sur membrane, lyophilisé et pesé.

100 μg d'extrait lyophilisé sont utilisés pour l'identification des ellagitanins majoritaires par un spectromètre de masse LSIMS.

1 mg de l'extrait est repris par du méthanol/eau (6:4) pour être analysée par HPLC couplée à un détecteur UV et à un spectromètre de masse LSIMS, selon le dispositif mis au point par Vivas et al. (1995).

La méthode de séparation HPLC est décrite dans le paragraphe suivant.

## 3. Dosage des composés phénoliques

### 3.1. Coumarines

Les coumarines sont quantitativement extraits par extraction à l'éther diéthylique à partir de 20 ml d'un extrait de bois. La phase organique est évaporée à sec et le résidu repris par du méthanol. L'analyse HPLC est réalisée par un Varian 5060 couplé avec un détecteur spectrofluorimètre (Kontron SFM23/B) et colonne Ultrasphère ODS. Les coumarines pures ont été fournies par Extrasynthèse (aesculine, aesculétine, scopolétine, ombelliférone, methyl-ombelliférone) et Sigma (sporalen). On observe les coumarines en fluorescence (excitation 425 nm et émission 325 nm).

### 3.2. Ellagitanins

#### 3.2.1. Produits de référence

La vescalagine et la castalagine sont isolées et purifiées à partir du duramen de Q. robur, dans les conditions décrites par Vivas et al. (1995). Les différentes roburines (A-E) et la grandinine proviennent de Scalbert (INA/INRA Thierval-Grignon).

#### 3.2.2. Séparation et dosage des ellagitanins par HPLC

La technique chromatographique de séparation et de dosage des ellagitanins est conforme à la méthode mise au point par Scalbert et al. (1990). Les extraits de bois sont analysés par HPLC sur colonne Ultrasphère ODS. La détection est conduite à λ=280 nm.

### 3.2.3. Dosage des ellagitanins totaux

a) Estimation du taux de composés phénoliques totaux

L'estimation de la richesse des extraits de bois en composés phénoliques totaux est réalisée soit par la méthode utilisant le réactif de Folin-Ciocalteu, soit par la mesure de l'absorbance à 280 nm des extraits dilués au 1/100 (Vivas et al., 1993). Ces deux méthodes donnent des résultats comparables mais non spécifiques des ellagitanins.

b) Réaction d'oxydation à l'acide nitreux

Dans cette méthode proposée par Bate-Smith (1972), les esters de l'acide hexahydroxyphénique et du glucose sont oxydés par l'acide nitreux sous azote. La réaction conduit à une coloration bleue que l'on mesure à 600 nm. Les résultats sont estimés en mg/g ou mg/l d'équivalent castalagine ($\varepsilon$600nm : 983 g$^{-1}$).

c) Dégradation acide

La méthode proposée est adaptée de celle mise au point par Peng et al. (1991). Elle est basée sur l'hydrolyse acide des ellagitanins, suivi d'un dosage par HPLC de l'acide ellagique libéré. Les résultats sont exprimés en mg/g d'équivalent castalagine, en considérant qu'une mole de castalagine donne dans ces conditions une mole d'acide ellagique (Peng et al., 1991).

4. Extraction et dosage des polysaccharides

4.1. Etude de la fraction polysaccharide des échantillons témoins et traités

4.1.1. Extraction des polysaccharides

139 g de sciure sèche sont mis à macérer 72 h, sur table d'agitation à température ambiante. La solution est ensuite filtrée puis centrifugée. L'extrait est alors concentré jusqu'à 500 ml.

4.1.2. Isolement des polysaccharides

Les extraits subissent 3 précipitations (une à 1:9 d'eau/éthanol à 95 % vol., deux à 1:5 du même mélange). La précipitation est conduite à 3° C pendant 12 h. Ensuite les fractions sont lyophilisées.

4.1.3. Caractérisation partielle

Le précipité présente un aspect floconneux de couleur très pâle, légèrement gris, qui est probablement un complexe avec les ellagitanins dont il est difficile d'isoler la fraction polysaccharidique.
4.1.4. Dosage par méthode chimique

   a) Dosage des polysaccharides neutres (Pn)
   Les polysaccharides neutres sont dosés par la méthode au phénol sulfurique. La densité optique est lue à 490 nm et les résultats sont exprimés en mg/l d'équivalent glucose.

   b) Dosage des polysaccharides acides (Pa)
   Les polysaccharides acides sont dosés par la méthode au métaphénylphénol. La densité optique est mesurée à 520 nm et les résultats sont exprimés en mg/l d'équivalent acide galacturonique.

4.2. Extraction et dosage des polysaccharides du bois

1 g de copeaux issus de chênes de différentes régions, dont le bois présente un grain caractéristique est mis à macérer 24 h dans l'eau à 20° C sur table d'agitation. La solution est collectée par filtration et centrifugée. La sciure récupérée est alors séchée et mise à macérer une nouvelle fois dans une solution de soude à 5 % dans les mêmes conditions. La solution alcaline est également collectée par filtration et centrifugée. Puis les deux catégories d'extraits (eau et soude) sont supplémentés en éthanol à 95 % à raison 1:5 en volume. Les polysaccharides sont alors collectés par centrifugation et repris par de l'eau distillée à 60° C. Le dosage des Pn est réalisé au phénol sulfurique et les Pa au métaphénylphénol. Les solutions de référence sont respectivement une solution aqueuse à 100 mg/l de glucose et 50 mg/l d'acide galacturonique pour Pn et Pa.

EXEMPLES

L'invention est illustrée par les exemples suivants, sans pour autant y être limitée.

Exemple 1

Dans une cuve de 15.000 litres d'eau, on immerge 8 piles d'environ 1 $m^3$ chacune de douelles de chêne superposées en quinconce, un espace étant ménagé entre chaque douelle pour permettre la circulation de la solution. La température de l'eau est ajustée à 40°C et le pH à 4,5-5,0, le bois étant laissé à tremper pendant 24 heures.
Ensuite, on ajoute 8 kg d'une formulation enzymatique comportant principalement :

- des cellulases (n° IUB EC 3.2.1) avec notamment

  - une activité mesurée en unité endocellulase (n° IUB EC 3.2.1.4) de 10.000 ECU/g de formulation,
  - une activité mesurée en unité xylanase (n° IUB EC 3.2.1.8) de 6.000 BXU/g de formulation, et
  - une activité mesurée en unité β-mannanase (n° IUB EC 3.2.1.78) de 4.000 MNU/g de formulation;

- des α-amylases (n° IUB EC 3.2.1.1) avec une activité mesurée en unité «α thermo-stable» de 10.000 αTU/g de formulation.

Ces concentrations sont bien entendu données à titre d'exemple. Il est clair qu'elles pourront varier considérablement en fonction des différents paramètres utilisés pour le traitement (temps de traitement, intensité désirée, nature du bois, volume du bain, volume de bois à traiter, pH et température du bain, etc.).

On laisse agir la solution enzymatique en maintenant une faible circulation afin d'assurer une répartition homogène des enzymes dans le bain.

Les bois sont ainsi traités pendant 48 heures puis sortis du bain et passés au séchoir pendant trois semaines pour leur permettre d'atteindre une humidité de 18% environ. Les douelles sont alors prêtes pour la fabrication de tonneaux, après un traitement total de 24 jours au lieu de 2 ans en cas de vieillissement naturel, pour une qualité organoleptique finale équivalente. Le séchage devra cependant être adapté à chaque lot de bois, afin d'éviter l'apparition de fentes.

Exemple 2

Selon le matériel et les méthodes ci-dessus, de la sciure et des blocs de chêne merrain (*Quercus petraea*) ont été traités avec une solution aqueuse d'enzymes suivant l'exemple 1 (1 g de préparation enzymatique dans 2,5 l d'eau par dm$^3$ de bois), pendant 24 h à 35-40°C et pH 5.

Après le traitement, les principaux groupes de composés du bois ont été fractionnés et les différentes fractions ont été comparées par rapport à un témoin non traité.

Pour réaliser ce fractionnement, l'extrait du bois de chêne est évaporé à sec. L'extrait sec obtenu est repris par un volume d'eau, sous agitation pendant 2 heures à 30°C. La fraction insoluble obtenue comporte les lignines. La fraction soluble est à nouveau évaporée à sec. L'extrait sec obtenu est alors repris par un volume d'un mélange éthanol/eau (9:1 en volume) et conservé 12 h à 4°C. La fraction insoluble obtenue comporte les polysaccharides. La fraction soluble quant à elle comporte les ellagitanins. Dans le bois traité, l'extrait sec est de 4,2% en poids par rapport au bois, contre 12,3% en poids pour le témoin de bois vert. Les pourcentages en poids de chacune des trois fractions par rapport à l'extrait sec sont repris dans le tableau I.

Tableau I

|  | Ellagitanins | Polysaccharides | Lignines |
|---|---|---|---|
| Témoin | 60% | 30% | 10% |
| Traité suivant l'invention | 35% | 50% | 15% |

Cet essai a permis de mettre en evidence une hydrolyse des ellagitanins, parallèlement avec une augmentation relative de la teneur en polysaccharides, dans l'extrait sec.

Ces différentes fractions ont ensuite été soumises à des dosages plus précis, mettant en évidence une diminution des matières sèches extractibles, des ellagitanins, des proanthocyanidines, et des coumarines glucosilées (voir tableau II).

Tableau II

| Résultats en mg/g de bois | Témoin | Traité suivant l'invention |
|---|---|---|
| Extrait sec sur : sciure | 123 | 42 |
| bois* | 73 | 26 |
| Ellagitanins sur : sciure | 54 | 21 |
| bois* | 32 | 14 |
| Proanthocyanidines sur : sciure | 0, 62 | 0,12 |
| bois* | 0, 47 | 0, 19 |
| Coumarines glucosylées (aesculine + scopoline) sur : | | |
| sciure | 5, 7 | 1,4 |
| bois* | 3,2 | 1,1 |
| aglucones (aesculétine + scopolétine) sur : | | |
| sciure | 1,3 | 6,8 |
| bois* | 4,8 | 7,1 |

* Dimension des échantillons : 5 cm x 5 cm x 10 cm

Une augmentation substantielle des polysaccharides a également été constatée dans un autre essai où le dosage a permis de distinguer entre les polysaccharides Pn et Pa (voir tableau III).

Tableau III

| | Polysaccharides solubles (mg/g de bois) | |
|---|---|---|
| | Neutres (Pn) | Acides (Pa) |
| Témoin | 370 | 25 |
| Traité suivant l'invention | 625 | 48 |

Le traitement permet donc d'éliminer certaines substances jugées indésirables de par leur caractère astringent (comme la castalagine) ou amer (comme l'aesculine) et de les transformer en molécules gustativement neutres (comme l'acide gallique, l'acide ellagique et dans une moindre mesure l'aesculétine), ainsi que d'augmenter la teneur en polysaccharides (ce qui tendra à donner plus de rondeur et de gras au vin élevé en présence de ce bois).

Une série de tests de dégustation ont également été réalisés (voir tableau IV). Ceux-ci confirment les expériences précédentes dans le sens où la quantité de bois nécessaire pour percevoir les caractères amers ou astringents sont beaucoup plus élevées dans le cas du bois traité enzymatiquement selon l'invention par rapport aux bois verts, séchés artificiellement ou naturellement.

Tableau IV

| Comparaison des seuils gustatifs de perception en fonction du mode d'affinage du bois, indiquant le seuil de perception gustatif ( $S_{50\%}$ )* en mg/l de solution modèle de vin | | |
|---|---|---|
| | Astringence | Amertume |
| Bois vert | 130 | 110 |
| Bois affiné<br><br>- selon l'invention<br>- par séchage naturel<br>- par séchage artificiel (séchoir) | <br><br>420<br>300<br>165 | <br><br>350<br>290<br>140 |

\* $S_{50\%}$ concentration à partir de laquelle une substance est perçue par 50 % des dégustateurs.

## Exemple 3

La mesure de l'activité enzymatique phénol-hétérosidase PeH est basée sur la libération de fonctions carboxyliques abaissant le pH du milieu de réaction.

Dans un tube à essai, on place 9 mg d'un extrait aqueux de chêne vert dans 9 ml d'une solution NaCl O,1 M, pH 4,5 et 1% de préparation enzymatique PE. Un tube témoin est réalisé dans les mêmes conditions, sans enzymes. Le pH est mesuré dans les deux tubes au temps 0 ($pHt_0$ pour le témoin et $pHe_0$ pour l'enzyme) et après 4 heures d'incubation à 25°C (respectivement $pHt_4$ et $pHe_4$). Les conditions optimales ont été établies au préalable et la corrélation entre la libération du glucose et la chute du pH sont parfaitement similaires. L'activité phénol-hétérosidase est calculée à partir de la relation :

$$PeH = [(pHt_0 - pHt_4) - (pHe_0 - pHe_4)/4] \times 100$$

(PeH étant exprimée en $10^{-3}$ unité de pH/ml/h)

Les mesures d'activité ont été réalisées sur différences espèces de chênes traités selon l'exemple 2, afin de déterminer les différences de performances éventuelles de la formulation enzymatique en fonction de l'espèce de chêne (voir tableau V).

Tableau V

| Activité phénol-hétérosidase | | | | | | | |
|---|---|---|---|---|---|---|---|
| PeH ($10^{-3}$ pH/ml/h) | Vosges | Allier | Limousin | Q.alba | Q.stellata | Q.lobata | Q.prinus |
| | 2, 5 | 2, 7 | 1, 9 | 4, 6 | 4, 3 | 4, 7 | 4, 9 |
| | 3,2 | 2,4 | 3,3 | 4,2 | 4,8 | 4,9 | 4,5 |
| | 2,8 | 3,6 | 3,5 | 3,9 | 4,3 | 4,5 | 4,7 |
| | 3,4 | 2, 2 | 2,4 | 4,5 | 4, 7 | 4, 6 | 4,7 |
| | 4,5 | 2,5 | 3,5 | 4,5 | 4,5 | 4,7 | 4,8 |
| Moyennes | 3,3 | 2,6 | 2,9 | 4,3 | 4,5 | 4,7 | 4 ,7 |

En ce qui concerne les chênes français (Vosges, Allier, Limousin), aucune différence significative n'est observée, la composition des extraits étant elle même souvent similaire.

Dans les espèces de chênes américains (Q. alba, stellata, lobata, prinus) on note que l'activité s'exprime de façon plus intense que dans les chênes français. Cela est sans doute dû à la présente de composés galloylés, présents en plus grandes quantités. L'activité étant plus intense, on peut donc s'attendre à un affinage plus important des espèces américaines, ce qui gustativement devrait les rapprocher des espèces françaises. Cette tendance est confirmée lors de dégustations.

Tableau VI

| Inhibition de l'activité phénol-hétérosidase par des composés phénoliques | | | | |
|---|---|---|---|---|
| PeH | Solution de référence d'ellagitanin (vescalagine) | avec des gallotanins à la concentration de | avec des procyanidines à la concentration de | |
| | 1 g/l | 1 g/l | 0,1 g/l | 0,5 g/l | 1 g/l |
| $10^{-3}$ pH /ml/h | 100 | 260 | 80 | 30 | 0 |

D'une façon générale, tous les substrats hétérosidiques sont utilisés par les activités enzymatiques présentes. Seuls les tanins condensés de raisin présentent une inhibition de l'activité phénol-hétérosidase. Cet effet est mis en évidence au tableau VI, par une diminution de PeH par rapport à la vescalagine.

Non seulement, la préparation enzymatique n'agit pas sur les procyanidines (tanins du raisin et du vin), mais en plus elle est fortement inhibée par ceux-ci. On peut en conclure qu'une éventuelle ( mais fort peu probable ) activité phénol-hétérosidase résiduelle dans le fût, n'altérera pas les qualités tanniques du vin développées par le raisin.

Les tanins condensés du raisin possèdent en fait la propriété de combiner les protéines, ce qui explique l'inactivation des activités de notre solution enzymatique. Cette inhibition est non réversible, car la solution enzymatique remise en absence de tanins condensés ne retrouve pas son activité initiale.

Tableau VII

| Influence de la concentration en ellagitanins sur l'activité phénol-hétérosidase | | | | | | |
|---|---|---|---|---|---|---|
| Concentration (g/l) | 1 | 1,5 | 2 | 3 | 4 | 5 |
| PeH ($10^{-3}$ pH/ml/h) | 2,7 | 2, 2 | 0,4 | 0,1 | 0,08 | 0 |

Sur des milieux trop riches en ellagitanins purs, l'activité est inhibée, comme l'indique le tableau VII. En pratique, lors du traitement du bois, ceci ne représente pas une limitation de la technique car la concentration locale en ellagitanins libres n'atteint jamais ces valeurs. Les ellagitanins du bois sont en effet associés à des structures glucosidiques qui les fixent sur les parois des fibres. La forme immobilisée de ces tanins ne présente, quant à elle, aucune inhibition sur l'activité phénol-hétérosidase. Au fur et à mesure de la digestion de ces structures par la préparation enzymatique, les ellagitanins sont libérés dans le milieu mais sont aussitôt hydrolysés par les enzymes présents.

De plus, l'application de la préparation enzymatique par trempage permet un lessivage du bois en appauvrissant les couches superficielles et diminuant ainsi d'autant la concentration locale en ellagitanins.

Tableau VIII

| Influence du pH sur l'activité phénol-hétérosidase | | | | | |
|---|---|---|---|---|---|
| pH | 2 | 3 | 4 | 5 | 6 |
| PeH ($10^{-3}$ pH/ml/h) | 0, 6 | 1,5 | 2,5 | 3,2 | 2,8 |

Comme l'indique le tableau VIII, l'activité phénol-hétérosidase est optimale à pH 5, ce qui correspond à l'optimum d'activité général de la préparation enzymatique, mesuré par la libération de glucose dans le milieu.

Tableau IX

| Valorisation du châtaignier par l'utilisation de la préparation enzymatique suivant l'invention | | |
|---|---|---|
| Résultats en mg/g | Témoin | |
| | Acide digallique | Coumarines glucosilées |
| Châtaignier Dordogne | 10,2 | 8,3 |
| Châtaignier Dauphiné | 9,3 | 5,7 |
| Châtaignier Gironde | 4,6 | 10,2 |
| | Traité suivant l'invention | |
| | Acide digallique | Coumarines glucosilées |
| Châtaignier Dordogne | < 0,1 | 2,3 |
| Châtaignier Dauphiné | 0,4 | 1,7 |
| Châtaignier Gironde | 0,8 | 3,5 |

Des extraits de châtaignier (riches en acide digallique) de diverses provenances ont été soumis (voir tableau IX) à une hydrolyse enzymatique par la préparation enzymatique selon l'exemple 2. Il en ressort une hydrolyse quasi totale de l'acide digallique en acide gallique, ainsi qu'une forte diminution des coumarines glucosilées (les coumarines aglucones étant insipides).

Ces résultats revêtent une grande importance car ils montrent la possibilité de valoriser une espèce peu employée à ce jour. Le châtaignier présente en effet toutes les qualités mécaniques requises pour faire du bois de tonnellerie, mais est très peu employé à cause de l'amertume importante qu'il procure.

Le traitement enzymatique réduit très fortement cette amertume et permet d'envisager une utilisation plus importante du châtaignier en tonnellerie, pour des usages particuliers où le chêne n'est pas approprié. Ainsi, pour l'élevage d'alcools blancs, le châtaignier est tout indiqué parce que ne possédant pas d'ellagitanins, il modifiera peu le goût de fruit de l'alcool, et ne possédant pas de pigments, il ne colorera pas non plus l'alcool mis à élever dans ces fûts.

## Revendications

1. Utilisation d'une composition enzymatique comprenant des enzymes de la clause IUB des cellulases pour l'affinage du bois vert, **caractérisée en ce que** ce bois est destiné au contact alimentaire.

2. Utilisation d'une composition enzymatique suivant la revendication 1, **caractérisée en ce que** le bois est constitué de duramen.

3. Utilisation d'une composition enzymatique suivant l'une quelconque des revendications précédentes, **caractérisée en ce que** le bois est choisi parmi les essences de chêne ou de châtaignier.

4. Utilisation d'une composition enzymatique suivant l'une quelconque des revendications précédentes, **caractérisée en ce que** le bois est destiné à la tonnellerie.

5. Utilisation d'une composition enzymatique suivant l'une quelconque des revendications précédentes, **caractérisée en ce que** cette composition contient en outre des enzymes choisies parmi les peroxydases, les lipases et les mélanges de ces enzymes.

6. Utilisation d'une composition enzymatique suivant l'une quelconque des revendications précédentes, **caractérisée en ce que** les enzymes de type cellulase sont choisies parmi les α-amylases (3.2.1.1), les cellobiohydrolases (3.2.1.91), les endocellulases (3.2.1.4), les endoglucanases (3.2.1.4), les β-glucosidases (3.2.1.21), les hémicellulases (3.2.1.4), les xylanases (3.2.1.8) et les β-mannanases (3.2.1.78).

7. Utilisation d'une composition enzymatique suivant l'une quelconque des revendications précédentes pour la modification des caractéristiques aromatiques des bois.

8. Utilisation d'un bain aqueux comportant des enzymes de la classe IUB des cellulases pour l'affinage du bois vert destiné au contact alimentaire.

9. Utilisation d'un bain aqueux suivant la revendication 8, **caractérisée en ce que** le bain présente une activité endocellulase comprise entre $1.10^6$ et $100.10^6$ ECU par $m^3$ de bois à traiter.

10. Utilisation d'un bain aqueux suivant l'une quelconque des revendications 8 ou 9, **caractérisée en ce que** le bain présente une activité xylanase comprise entre $0,6.10^6$ et $60.10^6$ BXU par $m^3$ de bois à traiter.

11. Utilisation d'un bain aqueux suivant l'une quelconque des revendications 8 à 10, **caractérisée en ce que** le bain présente une activité β-mannanase comprise entre $0,4.10^6$ et $40.10^6$ MNU par $m^3$ de bois à traiter.

12. Utilisation d'un bain aqueux suivant l'une quelconque des revendications 8 à 11, **caractérisée en ce que** le bain présente une activité α-amylase comprise entre $1.10^6$ et $100.10^6$ αTU par $m^3$ de bois à traiter.

13. Procédé d'affinage du bois vert destiné au contact alimentaire **caractérisé en ce qu'**il comporte une étape de mise en contact du bois avec un bain comportant des enzymes de la classe IUB des cellulases.

14. Procédé suivant la revendication 13, **caractérisé en ce qu'**il comporte une étape ultérieure de séchage artificiel.

15. Procédé suivant l'une quelconque des revendications 13 ou 14, **caractérisé en ce que** la mise en contact du bois avec le bain est effectuée par immersion du bois durant une période variant de 30 minutes à 2 semaines.

16. Procédé suivant l'une quelconque des revendications 13 à 15, **caractérisé en ce que** le pH du bain est compris entre 3 et 8.

17. Procédé suivant l'une quelconque des revendications 13 à 16, **caractérisé en ce que** la température du bain est comprise entre 20 et 70°C.

18. Procédé suivant l'une quelconque des revendications 13 à 17, **caractérisé en ce que** l'humidité du bois à traiter est ajustée entre 25 et 45% avant de soumettre le bois à la préparation enzymatique.

**Claims**

1. Use of an enzymatic composition comprising enzymes of the IUB cellulase class for seasoning green wood, **characterized in that** this wood is intended to come into contact with food.

2. Use of an enzymatic composition according to claim 1, **characterized in that** the wood consists of duramen.

3. Use of an enzymatic composition according to any one of the preceding claims, **characterized in that** the wood is chosen from species of oak or of chestnut.

4. Use of an enzymatic composition according to any one of the preceding claims, **characterized in that** the wood is intended for cooperage.

5. Use of an enzymatic composition according to any one of the preceding claims, **characterized in that** this composition also contains enzymes chosen from peroxidases, lipases and mixtures of these enzymes.

6. Use of an enzymatic composition according to any one of the preceding claims, **characterized in that** the enzymes of the cellulase type are chosen from α-amylases (3.2.1.1), cellobiohydrolases (3.2.1.91), endocellulases (3.2.1.4), endoglucanases (3.2.1.4), β-glucosidases (3.2.1.21), hemicellulases (3.2.1.4), xylanases (3.2.1.8) and β-mannanases (3.2.1.78) .

7. Use of an enzymatic composition according to any one of the preceding claims, for modifying the aromatic characteristics of wood.

8. Use of an aqueous bath comprising enzymes of the IUB cellulase class for seasoning green wood intended to come into contact with food.

9. Use of an aqueous bath according to claim 8, **characterized in that** the bath has an endocellulase activity of between $1 \times 10^6$ and $100 \times 10^6$ ECU per m$^3$ of wood to be treated.

10. Use of an aqueous bath according to either of Claims 8 and 9, **characterized in that** the bath has a xylanase activity of between $0.6 \times 10^6$ and $60 \times 10^6$ BXU per m$^3$ of wood to be treated.

11. Use of an aqueous bath according to any one of Claims 8 to 10, **characterized in that** the bath has a β-mannanase activity of between $0.4 \times 10^6$ and $40 \times 10^6$ MNU per m$^3$ of wood to be treated.

12. Use of an aqueous bath according to any one of Claims 8 to 11, **characterized in that** the bath has an α-amylase activity of between $1 \times 10^6$ and $100 \times 10^6$ $_\alpha$TU per m$^3$ of wood to be treated.

13. Method for seasoning green wood intended to come into contact with food, **characterized in that** it comprises a step of bringing the wood into contact with a bath comprising enzymes of the IUB cellulase class.

14. Method according to claim 13, **characterized in that** it comprises a subsequent step of artificial drying.

15. Method according to either of claims 13 and 14, **characterized in that** the wood is brought into contact with the bath by immersing the wood for a period of time ranging from 30 minutes to 2 weeks.

16. Method according to any one of claims 13 to 15, **characterized in that** the pH of the bath is between 3 and 8.

17. Method according to any one of claims 13 to 16, **characterized in that** the temperature of the bath is between 20 and 70°C.

18. Method according to any one of claims 13 to 17, **characterized in that** the water content of the wood to be treated is adjusted to between 25 and 45% before subjecting the wood to the enzymatic preparation.

**Patentansprüche**

1. Verwendung einer Enzymzusammensetzung, die Enzyme der Cellulasen-Klasse IUB enthält, zur Veredelung von frischem Holz, **dadurch gekennzeichnet, daß** dieses Holz zum Kontakt mit Nahrungsmitteln bestimmt ist.

2. Verwendung einer Enzymzusammensetzung nach Anspruch 1, **dadurch gekennzeichnet, daß** das Holz aus Kernholz besteht.

3. Verwendung einer Enzymzusammensetzung nach einem der vorhergehenden Ansprüche, **dadurch gekennzeichnet, daß** das Holz aus der Gruppe Eichenund Kastanienarten stammt.

4. Verwendung einer Enzymzusammensetzung nach einem der vorhergehenden Ansprüche, **dadurch gekennzeichnet, daß** das Holz für die Böttcherei bestimmt ist.

5. Verwendung einer Enzymzusammensetzung nach einem der vorhergehenden Ansprüche, **dadurch gekennzeichnet, daß** die Zusammensetzung weiterhin Enzyme aus der Gruppe der Peroxidasen, Lipasen sowie Mischungen dieser Enzyme enthält.

6. Verwendung einer Enzymzusammensetzung nach einem der vorhergehenden Ansprüche, **dadurch gekennzeichnet, daß** die Enzyme des Cellulase-Typs aus der Gruppe der α-Amylasen (3.2.1.1), Cellobiohydrolasen (3.2.1.91), Endocellulasen (3.2.1.4), Endoglucanasen (3.2.1.4), β Glucosidasen (3.2.1.21), Hemicellulasen (3.2.1.4), Xylanasen (3.2.1.8) und β-Mannanasen (3.2.1.78) stammen.

7. Verwendung einer Enzymzusammensetzung nach einem der vorhergehenden Ansprüche zur Modifizierung der Aromaeigenschaften von Hölzern.

8. Verwendung eines wäßrigen Bads, das Enzyme der Cellulasen-Klasse IUB enthält, zur Veredelung von frischem Holz, das zum Kontakt mit Nahrungsmitteln bestimmt ist.

9. Verwendung eines wäßrigen Bads nach Anspruch 8, **dadurch gekennzeichnet, daß** das Bad eine Endocellulaseaktivität zwischen $1x10^6$ und $100x10^6$ ECU pro $m^3$ zu behandelndem Holz aufweist.

10. Verwendung eines wäßrigen Bads nach Anspruch 8 oder 9, **dadurch gekennzeichnet, daß** das Bad eine Xylanaseaktivität zwischen $0{,}6x10^6$ und $60x10^6$ BXU pro $m^3$ zu behandelndem Holz aufweist.

11. Verwendung eines wäßrigen Bads nach einem der Ansprüche 8 bis 10, **dadurch gekennzeichnet, daß** das Bad eine β-Mannanaseaktivität zwischen $0{,}4x10^6$ und $40x10^6$ MNU pro $m^3$ zu behandelndem Holz aufweist.

12. Verwendung eines wäßrigen Bads nach einem der Ansprüche 8 bis 11, **dadurch gekennzeichnet, daß** das Bad eine α-Amylaseaktivität zwischen $1x10^6$ und $100x10^6$ α-TU pro $m^3$ zu behandelndem Holz aufweist.

13. Verfahren zur Veredelung von frischem Holz, das zum Kontakt mit Nahrungsmitteln bestimmt ist, **dadurch gekennzeichnet, daß** es einen Schritt umfaßt, bei dem Holz mit einem Bad, das Enzyme der Cellulase-Klasse IUB enthält, in Kontakt gebracht wird.

14. Verfahren nach Anspruch 13, **dadurch gekennzeichnet, daß** es einen weiteren Schritt mit einer künstlichen Trocknung umfaßt.

15. Verfahren nach Anspruch 13 oder 14, **dadurch gekennzeichnet, daß** das Holz einer Tauchbehandlung in dem Bad über einen Zeitraum im Bereich von 30 Minuten bis 2 Wochen unterzogen wird.

16. Verfahren nach einem der Ansprüche 13 bis 15, **dadurch gekennzeichnet, daß** der pH-Wert des Bads zwischen 3 und 8 beträgt.

17. Verfahren nach einem der Ansprüche 13 bis 16, **dadurch gekennzeichnet, daß** die Badtemperatur zwischen 20 und 70°C beträgt.

18. Verfahren nach einem der Ansprüche 13 bis 17, **dadurch gekennzeichnet, daß** vor der Behandlung des Holzes mit dem Enzympräparat der Feuchtigkeitsgehalt des zu behandelnden Holzes auf 25 bis 45% gebracht wird.